# EUROPEAN PATENT APPLICATION

(11) **EP 4 495 122 A1**
(43) Date of publication of application: **22.01.2025**
(21) Application number: 22945613.2
(22) Date of filing: 14.12.2022
(51) Int. Cl.: C07D 493/18, A61K 31/357, A61P 27/02, A61P 29/00

(54) **ARTEMISININ DERIVATIVES, PREPARATION METHOD THEREFOR, AND USES THEREOF**

(30) Priority: 10.06.2022 CN 202210652732
(71) Applicant: Shanghai Innofortune Boitech Co. Ltd., Pudong, Shanghai 201306 (CN)
(72) Inventor: WANG, Yandong, Guangzhou, Guangdong 510600 (CN); LIU, Guoqiang, Shijiazhuang, Hebei 050000 (CN); LIU, Wei, Shanghai 210516 (CN)
(74) Representative: Novagraaf International SA
(86) International application number: PCT/CN2022/138838
(87) International publication number: WO 2023/236485

(57) **Abstract**

Provided are an artemisinin derivative and a preparation method and use thereof. A structural formula of the artemisinin derivative is shown in Formula I. The compound has suitable solubility and is relatively stable under storage conditions. Pharmacokinetic experiments on the compound have shown that artemisinin is detectable in ocular tissues and is present in cornea, conjunctiva, and aqueous humor at a relatively high drug concentration. The compound is therefore an excellent precursor to artemisinin. The compound shown in Formula I exhibits remarkable absorbability, and the drug concentration in the ocular tissues indicates that the compound is well absorbed. The compound exhibits significantly better inhibitory effects on neovascularization in burn models than artemisinin, and can be prepared into liquid formulations such as eye drops, injection, etc., for treatment of ocular tissue diseases.

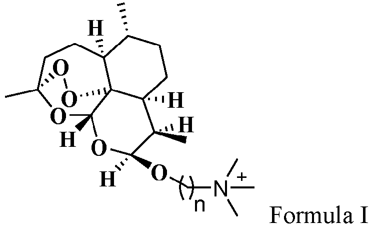

## Description

### Field of the Invention

The present invention belongs to the field of medicine, and in particular relates to a derivative of artemisinin, and a preparation method and use thereof.

### Background of the Invention

Artemisinin is the best drug for treating drug-resistant malaria, and artemisinin-based combination therapies are the most effective and critical means currently used for treating malaria. However, in recent years, with the deepening of research, other effects of artemisinin, such as its anti-tumor, pulmonary hypertension treating, anti-diabetic, embryotoxicity, anti-fungal, immune regulation effects and so on have been continually discovered, used and studied.

Malaria is an insect-borne infectious disease caused in humans bitten by malaria parasite-infected Anopheles mosquitoes. After multiple malaria attacks that last long, a person can develop hepatosplenomegaly, accompanied by symptoms such as anemia. In making it possible to treat malaria to some extent, the contribution of artemisinin cannot go unnoticed. The peroxy bond in the structure of artemisinin exhibits oxidizability and is an essential group in fighting against malaria. The mechanism of action is that free radicals produced by artemisinin in the body bind to proteins in the malaria parasite, changing the cell membrane structure of the malaria parasite. The binding of the free radicals to the proteins in the malaria parasite can cause the double membrane of the mitochondria to swell and crack and eventually fall off, resulting in damage to the structure and functions of cells of the malaria parasite, and also to some extent affecting the chromatin in the nucleus. On the other hand, amino acids are basic substances that make up proteins; after the action of artemisinin, uptake of isoleucine by the malaria parasite is reduced, which impairs synthesis of proteins in the malaria parasite. Artemisia annua not only can kill pathogens, but also has anti-schistosomiasis, toxoplasma gondii infection treating, anti-pneumocystis carinii, and anti-coccidiosis effects and so on. Clinical trials have proved that artemisinin and its derivatives have not been found to have particularly significant side effects in the treatment of malaria.

In recent years, artemisinin has been found to have certain effects on the treatment of retinal macular degeneration and fundus diseases. However, artemisinin has physical and chemical properties of being instable, easily oxidized, and insoluble in water and therefore is difficult to be used in liquid formulations, especially in the form of eye drops. Both artemisinin and artesunate are hardly soluble in water and can hardly be absorbed by ocular tissues. It is therefore very necessary to provide an artemisinin derivative that has suitable solubility and can be absorbed into ocular tissues.

### Summary of the Invention

An objective of the present invention is to provide a compound shown in Formula I or a pharmaceutically acceptable salt thereof. The compound is an excellent derivative of artemisinin.

To achieve the above objective, the present invention adopts the following technical solutions.

A compound shown in Formula I or a pharmaceutically acceptable salt thereof:

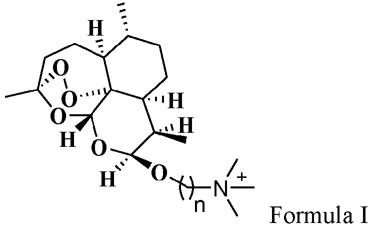

In Formula I, n may be an integer from 1 to 15, for example 2, 3, or 4.

Further, the compound shown in Formula I may be a compound shown in Formula II:

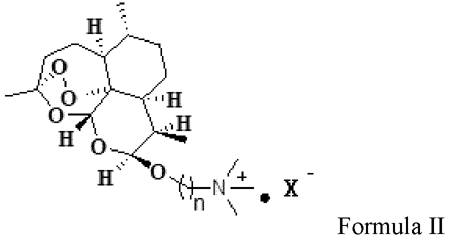

In Formula II, n may be an integer from 1 to 15, for example 2, 3, or 4; and X⁻ represents an anion.

Further, X⁻ is selected from acid anions produced when the following acids are ionized: hydrochloric acid, sulfuric acid, phosphoric acid, HBr, HI, citric acid, fumaric acid, succinic acid, acetic acid, nitric acid, hydrogen sulfate, lactic acid, methanesulfonic acid, and benzenemethanesulfonic acid.

The pharmaceutically acceptable salt of the compound shown in Formula I of the present invention refers to a salt which is, within the scope of sound medical judgment, suitable for use in contact with the tissues of humans and lower animals without undue toxicity, irritation, allergic response, and the like, in keeping with a reasonable benefit/risk ratio.

Preferably, the pharmaceutically acceptable salt of the compound shown in Formula I is citrate, transbutenedioate, salicylate, L-tartrate, fumarate, hydrochloride, ethanoate, nitrate, sulfate, bisulfate, phosphate, biphosphate, acetate, oxalate, lactate, lysine or aspartate.

The present invention also provides a method for preparing the above described compound shown in Formula I.

The method for preparing the compound shown in Formula I provided in the present inventioncomprises the following steps:
1) dissolving 3-dimethylamino-1-alkyl alcohol in acetonitrile, and adding iodomethane drop-wise, followed by stirring for a reaction to obtain a quaternary ammonium salt intermediate; and
2) dispersing dihydroartemisinin in dichloromethane, followed by successively adding the quaternary ammonium salt intermediate and an catalytic amount of boron trifluoride ether for a reaction to obtain the compound shown in Formula I.

The alkyl group in 3-dimethylamino-1-alkyl alcohol may be propyl, butyl, etc.

The ratio of dose by mass of dimethylamino-alkyl alcohol to iodomethane can be 1: (3-5); and the ratio of dose by mass of dihydroartemisinin to the quaternary ammonium salt intermediate can be 1: (1-2).

Preferably, the reaction is conducted at a temperature of 20-45°C, for a time period of 2-6 hours.

The present invention also provides use of the above-described compound shown in Formula I or its pharmaceutically acceptable salt in preparation of a drug for prevention and/or treatment of an ocular tissue disease.

The present invention also provides use of the above-described compound shown in Formula I or its pharmaceutically acceptable salt in preparation of an inhibitor for prevention and/or treatment of corneal neovascularization.

The present invention provides a drug or a pharmaceutical composition for prevention and/or treatment of an ocular tissue disease, which comprises the above-described compound shown in Formula I or its pharmaceutically acceptable salt, and a pharmaceutically acceptable carrier.

The drug may be introduced into, for example, a muscle, an intradermal tissue, a subcutaneous tissue, a vein, a mucosal tissue of an organism by injection, spraying, nose drops applying, eye drops applying, penetration, absorption, and a physical or chemically mediated method; or introduced into an organism by being mixed with or wrapped by another substance.

Preferably, the ocular tissue disease is ocular tissue inflammation or ocular tissue macular degeneration.

Preferably, the pharmaceutically acceptable carrier comprises at least one selected from diluents, excipients, fillers, binders, humectants, disintegrants, absorption accelerators, surfactants, adsorption carriers, and lubricants.

Preferably, the pharmaceutical or the pharmaceutical composition is in the form of a liquid formulation. Preferably, the liquid formulation is eye drops or injection. Preferably, the eye drops have a pH value of 5.5-6.5.

The present invention brings the following beneficial effects. The compound shown in Formula I of the present invention has suitable solubility and is relatively stable under storage conditions, and is an excellent precursor to artemisinin. The compound shown in Formula I of the present invention is significantly advantageous in its absorbability, and can be prepared into liquid formulations such as eye drops, injections, etc., for the treatment of ocular tissue diseases.

### Brief Description of the Drawings

Fig. 1 shows photographs for fluorescein staining in each of treatment groups in Example 6; and
Fig. 2 shows photographs for neovascularization in each of treatment groups in Example 6.

### Detailed Description of the Embodiments

The present invention is further elaborated below in conjunction with specific embodiments. The present invention, however, is not limited to the following embodiments. Methods described are all conventional methods unless otherwise specified. Raw materials described are all commercially available unless otherwise specified.

### Example 1: Synthesis of SZY1905-P30

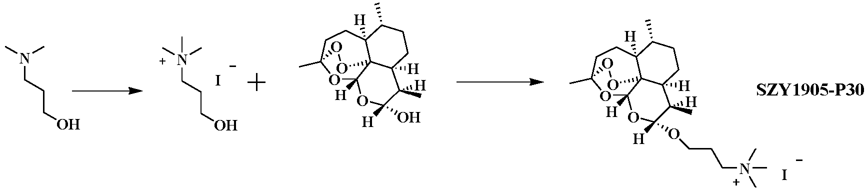

5 g of 3-dimethylamino-1-propanol was added to a 100-mL single-neck flask, followed by adding 100 mL of acetonitrile and stirring for dissolution. 20.7 g of iodomethane was then added drop-wise. After the addition, the resulting solution was stirred at room temperature for 2 hours, and TLC was used to determine that a reaction of the raw materials had gone to completion. 100 mL of methyl tert-butyl ether was added drop-wise to the reaction solution, and a white solid precipitated out of the solution. Vacuum filtration was then performed, obtaining 10.4 g of quaternary ammonium salt intermediate I.

10 g of quaternary ammonium salt intermediate I and 8.0 g of dihydroartemisinin were added successively to a 100-mL single-neck flask, followed by adding 100 mL of dichloromethane and stirring for dispersion. A catalytic amount of boron trifluoride ether solution was then added drop-wise. After the addition, the clear solution gradually turned dark red. The solution was stirred at room temperature for 2 hours, and TLC was used to determine that a reaction of the raw materials had gone to completion. The reaction solution was then washed with saturated ammonium chloride and saturated sodium chloride in turn. The resulting organic phase was dried in a rotary drier under reduced pressure, obtaining 10.8 g of a crude product of SZY1905-P30. The crude product was purified by dichloromethane:methanol (1:0-30:1) column chromatography, obtaining 1.4 g of foamy solid SZY1905-P30.

1HNMR CDCl₃ δ: 5.4 (s, 1H), 5.3 (m, 1H), 5.0 (m, 1H), 4.6-4.4 (m, 2H), 3.7-3.4 (m, 12H), 1.9-1.8 (m, 3H), 1.6-1.3 (m, 12H), 1.1 (m, 3H), 0.8 (m, 3H).

LC-MS: m/z = 384.3 (M+1).

### Example 2: Synthesis of SZY1905-P31

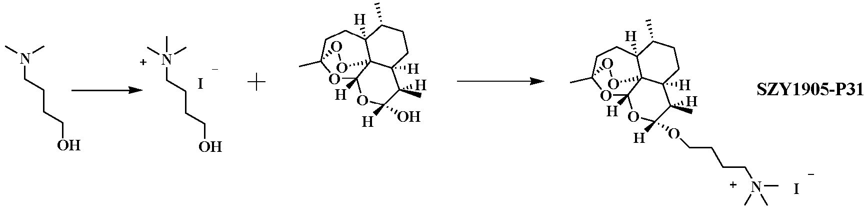

1 g of 3-dimethylamino-1-butanol was added to a 100-mL single-neck flask, followed by adding 20 mL of acetonitrile and stirring for dissolution. 3.64 g of iodomethane was then added drop-wise. After the addition, the resulting solution was stirred at room temperature for 2 hours, and TLC was used to determine that a reaction of the raw materials had gone to completion. 20 mL of methyl tert-butyl ether was added drop-wise to the reaction solution, and a white solid precipitated out of the solution. Vacuum filtration was then performed, obtaining 2.0 g of quaternary ammonium salt intermediate II.

2.0 g of quaternary ammonium salt intermediate II and 2.0 g of dihydroartemisinin were added successively to a 100-mL single-neck flask, followed by adding 40 mL of dichloromethane and stirring for dispersion. A catalytic amount of boron trifluoride ether was then added drop-wise. After the addition, the clear solution gradually turned dark red. The solution was stirred at room temperature for 2 hours, and TLC was used to determine that a reaction of the raw materials had gone to completion. The reaction solution was then washed with saturated ammonium chloride and saturated sodium chloride in turn. The resulting organic phase was dried in a rotary drier under reduced pressure, obtaining 2.5 g of a crude product of SZY1905-P31. The crude product was purified by dichloromethane:methanol (1:0-30:1) column chromatography, obtaining 300 mg of foamy solid SZY1905-P31.

1HNMR CDCl₃ δ: 5.4 (s, 1H), 5.0 (m, 1H), 3.8 (m, 1H), 3.6-3.5 (m, 4H), 3.3 (m, 10H), 2.2 (m, 1H), 2.0 (m, 1H), 1.9-1.8 (m, 4H), 1.6-1.3 (m, 11H), 1.1 (m, 3H), 0.8 (m, 3H).

LC-MS: m/z = 398.1.0 (M+1).

### Example 3: Synthesis of a maleate compound

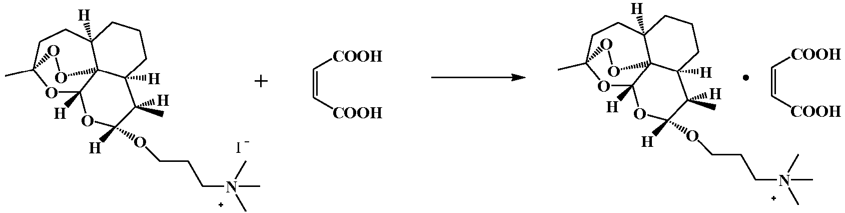

1 g of SZY1905-P30 was added to a 100-mL single-neck flask, followed by adding 20 mL of ethanol and stirring for dissolution. 0.32 g of maleic acid in 3 mL of ethanol solution was then added drop-wise. After the addition, the resulting solution was cooled to 0°C in an ice bath, and a white solid gradually precipitated. The solution was stirred at 0°C for 2 hours and then subjected to vacuum filtration, obtaining 0.8 g of a maleate compound.

1HNMR d6-DMSO δ: 6.2 (s, 2H), 5.5 (s, 1H), 5.3 (d, 1H), 3.6-3.5 (m,2H), 3.3 (m,1H), 2.9 (s, 6H), 2.2-2.0 (m,4H), 1.9-1.8 (m, 5H), 1.7-1.3 (m, 9H), 1.2 (m, 3H), 0.9 (m, 3H).

LC-MS: m/z=384.3(M+1).

Other organic salts were obtained in a similar way by the above method with maleic acid being replaced with other organic acids.

### Example 3: Testing of solubility at different pH values

First, a series of solutions with pH=5-8 were prepared using phosphoric acid and NaOH. Preparation methods were shown in Table 1.

**Table 1 Preparation methods of solutions with different pH values**

| | Preparation methods |
|---|---|
| Purified water | Self-made |
| pH=5.0 | Obtained by diluting an aqueous solution of phosphoric acid |
| pH=6.0 | Obtained by diluting an aqueous solution of phosphoric acid |
| pH=7.0 | A buffer solution prepared using KH₂PO₄ and NaOH |
| pH=8.0 | A buffer solution prepared using KH₂PO₄ and NaOH |

The solubility of SZY1905-P30 and SZY1905-P31 at different pH values is shown in Table 2.

**Table 2 Solubility of SZY1905-P30 and SZY1905-P31 at different pH values at 25°C (room temperature)**

| pH values | SZY1905-P30 (mg/mL) | SZY1905-P31 (mg/mL) |
|---|---|---|
| Purified water | >10 | >10 |
| 5.0 | >1 | >1 |
| 6.0 | >1 | >1 |
| 7.0 | >1 | >1 |
| 8.0 | >1 | >1 |

### Example 4: Testing of stability at different pH values

### 1. Stability of SZY1905-P30 at different pH values

10 mg of the sample was weighed and 10 mL of an aqueous solution at a different pH value was added, followed by ultrasonic dissolving and filtration. HPLC testing was then performed. Results are shown in Table 3.

**Table 3 Stability of SZY1905-P30 at different pH values**

| | 0 day (%) | 8^{th} day (%) | 20^{th} day (%) | 30^{th} day (%) |
|---|---|---|---|---|
| Purified water | 97.09 | 96.91 | 97.04 | 97.28 |
| pH=5.0 | 97.23 | 96.90 | 97.01 | 97.25 |
| pH=6.0 | 97.24 | 96.95 | 96.81 | 97.23 |
| pH=7.0 | 97.34 | 97.09 | 96.97 | 97.26 |
| pH=8.0 | 97.22 | 97.13 | 96.98 | 97.13 |

### 2. Stability of SZY1905-P31 at different pH values

10 mg of the sample was weighed and 10 mL of an aqueous solution at a different pH value was added, followed by ultrasonic dissolving and filtration. HPLC testing was then performed. Results are shown in Table 4.

**Table 4 Stability of SZY1905-P31 at different pH values**

| | 0 day (%) | 8^{th} day (%) | 20^{th} day (%) | 30^{th} day (%) |
|---|---|---|---|---|
| Purified water | 98.05 | 97.68 | 97.35 | 97.46 |
| pH=5.0 | 98.11 | 97.69 | 97.38 | 97.48 |
| pH=6.0 | 98.16 | 97.64 | 97.39 | 97.48 |
| pH=7.0 | 98.26 | 97.85 | 97.57 | 97.58 |
| pH=8.0 | 98.14 | 97.64 | 97.33 | 97.49 |

### Example 5: Synthesis of a sulfate compound of SZY1905-P30

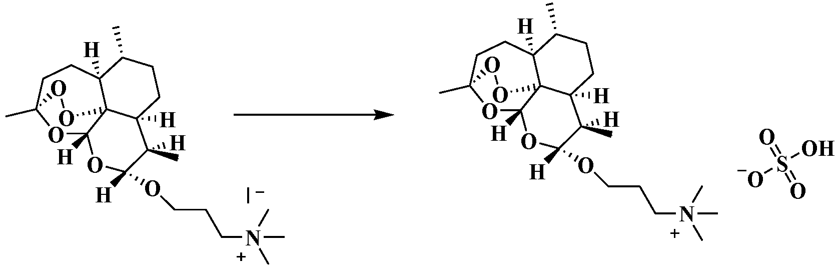

3.5 g of SZY1905-P30 was added to a 100-mL single-neck flask, followed by adding 35 mL of ethanol and stirring for dissolution. The resulting solution was cooled in an ice bath. 1.1 g of 30% sodium hydroxide solution was added drop-wise, followed by stirring for 10 minutes and then drop-wise addition of 2.8 mL of 3N H₂SO₄ ethanol solution. A white solid gradually precipitated. The solution was stirred at 0°C for 2 hours and then subjected to vacuum filtration, obtaining 1.6 g of a sulfate compound of SZY1905-P30.

1HNMR d6-DMSO δ: 5.8 (s, 1H), 5.5 (d, 1H), 3.6-3.4 (m, 2H), 3.3 (m, 1H), 3.0 (s, 9H), 2.6- 2.2 (m, 5H), 2.2-1.8 (m, 4H), 1.7-1.4 (m, 9H), 1.2 (m, 3H), 0.9 (m, 3H).

LC-MS: m/z=384.3.

A sulfate compound of SZY1905-P31 was obtained in a similar way by the above method with artemisinin quaternary ammonium propyl ether iodate (SZY1905-P30) being replaced with artemisinin quaternary ammonium butyl ether iodate (SZY1905-P31).

Other salts of SZY1905-P30 and SZY1905-P31 could also be obtained in a similar way.

### Comparative Example 1: Synthesis of a hydrochloride compound of SZY1905-P32

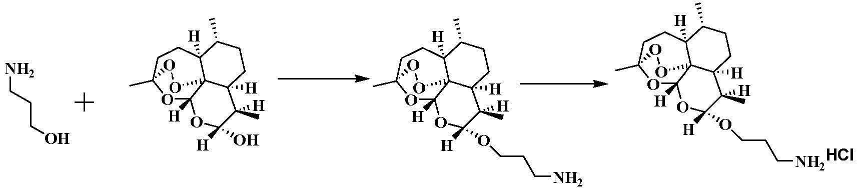

3.0 g of 3-amino-1-propanol and 6.0 g of dihydroartemisinin were added successively to a 100-mL single-neck flask, followed by adding 40 mL of dichloromethane and stirring for dispersion. A catalytic amount of boron trifluoride ether was added drop-wise. After the addition, the clear solution gradually turned dark red. The solution was stirred at room temperature for 2 hours, and TLC was used to determine that a reaction of the raw materials had gone to completion. The reaction solution was washed with saturated ammonium chloride and saturated sodium chloride in turn. The resulting organic phase was dried in a rotary drier under reduced pressure, obtaining a crude product. The crude product was purified by dichloromethane:methanol (1:0-10:1) column chromatography, obtaining 2.3 g of foamy SZY1905-P32.

2.3 g of SZY1905-P32 was added to a 100-mL single-neck flask, followed by adding 20 mL of ethanol and stirring for dissolution. The resulting solution was cooled in an ice bath. 2.5 mL of 3N HCl ethanol solution was added drop-wise. After the addition, a white solid gradually precipitated. The solution was then stirred at 0°C for 2 hours and subjected to vacuum filtration, obtaining 1.8 g of a hydrochloride compound of SZY1905-P32.

1HNMR d6-DMSO δ: 5.4 (s, 1H), 5.0 (d, 1H), 3.9-3.5 (m, 4H), 3.3 (m, 2H), 2.5-2.1 (m, 5H), 1.9-1.8 (m, 3H), 1.6-1.1 (m, 9H), 1.0 (m, 3H), 0.9 (m, 3H).

LC-MS: m/z = 342.2(M+1).

### Example 6 Effects on corneal neovascularization (formation of new blood vessels in cornea) in New Zealand rabbits with alkali burn

### I. Objective of the experiment

Effects of eye drops containing 1% SZY1905-P30, 1% SZY1905-P31, and 1% SZY1905-P32 on corneal neovascularization in New Zealand rabbits with alkali burn were to be observed.

### II. Experimental materials, instruments, and animals

### 1. Experimental materials

0.5% tetracaine hydrochloride (Zhongshan Ophthalmic Center, Sun Yat-sen University; 20220113); Tobramycin dexamethasone sodium phosphate eye drops (Zhongshan Ophthalmic Center, Sun Yat-sen University; 20211217); chloralhydrate solution (Zhongshan Ophthalmic Center, Sun Yat-sen University; 20220224); 1.0 mol/L NaOH solution; filter paper; stopwatch; Sodium fluorescein eye drops (Zhongshan Ophthalmic Center, Sun Yat-sen University); 0.9% Sodium chloride injection (Guangdong Yixiang Pharmaceutical Co., Ltd., 210926202); 50-mL syringes; 1-mL syringes.

### 2. Experimental instruments

Digital slit-lamp microscope BX-900 (Switzerland; Haag-Streit Gruppe); digital scale

3. 1% SZY1905-P30 (22030201; 5.0 mL/each); 1% SZY1905-P30 (22030202; 5.0 mL/each); 1% SZY1905-P32 (22031501; 5.0 mL/each).

### 4. Experimental animals

Healthy New Zealand rabbits, purchased from Huadong Xinhua Experimental Animal Farm, Huadu District, Guangzhou, anterior segment lesions excluded by slit-lamp examination, aged 2.0-2.5 months, and weighed 2.2-2.9 kg.

### III. Process of the experiment

### 1. Weighing of experimental animals: The experimental animals were weighed with the digital scale, and the weights were recorded.

| Animal No. | Weight (kg) | Animal No. | Weight (kg) |
|---|---|---|---|
| 71 | 2.6 | 117 | 2.6 |
| 73 | 2.2 | 118 | 2.8 |
| 86 | 2.4 | 143 | 2.4 |
| 87 | 2.3 | 145 | 2.9 |
| 89 | 2.4 | 147 | 2.4 |
| 93 | 2.4 | 148 | 2.7 |
| 94 | 2.4 | 151 | 2.8 |
| 97 | 2.3 | 156 | 2.7 |
| 101 | 2.2 | 157 | 2.6 |
| 108 | 2.7 | 161 | 2.8 |
| 109 | 2.7 | 166 | 2.7 |
| 110 | 2.3 | 168 | 2.9 |

### 2. Experimental animal modeling

Twenty-four New Zealand rabbits were first given intravenous anesthesia with a chloralhydrate solution at a dose of about 0.5 mL/kg, followed by ocular surface anesthesia with 0.5% tetracaine hydrochloride eye drops. A piece of filter paper with a diameter of about 8 mm was immersed in 1 mol/L NaOH solution for 1 minute to reach saturation. The saturated filter paper was placed in the center of the cornea for 1 minute and then removed, immediately followed by repeatedly rinsing the cornea and the conjunctival sac with 0.9% sodium chloride injection for 1 minute. After that, tobramycin dexamethasone sodium phosphate eye drops were administered.

### 3. Administration of eye drops to experimental animals

After modeling, the twenty-four animals were divided into four groups, which were: Model group; SZY1905-P30 eye drops-administered group; SZY1905-P31 eye drops-administered group; 1% SZY1905-P32 eye drops-administered group. The eye drops were administered 3 times a day, 2 drops each time. The Model group was not administered with any drugs.

| Group | Animal No. |
|---|---|
| Model group | 71, 73, 110, 117, 118, 143 |
| SZY1905-P30 eye drops-administered group | 86, 89, 93, 145, 148, 166 |
| SZY1905-P31 eye drops-administered group | 87, 94, 97, 147, 151, 168 |
| SZY1905-P32 eye drops-administered group | 101, 108, 109, 156, 157, 161 |

### 4. Photographing with the digital slit lamp

Photographs were taken with the digital slit lamp respectively before the modeling, 1 day after the modeling (0 day of administration), 7 days after the modeling (6^{th} day of administration), and 14 days after the modeling (13^{th} day of administration). The cornea was then stained with fluorescein sodium, and observed under the slit-lamp microscope and photographed.

### 4. Experimental results

New Zealand rabbit No. 157 died of tachypnea on the 5^{th} day after the modeling, and a total of five animals (101, 108, 109, 156, 161) were left in the 1% SZY1905-P32 eye drops-administered group, with 10 eyes for subsequent experiments.

### (1) Eye inflammation

Number of New Zealand rabbit eyes with conjunctival congestion, corneal edema, neovascularization, and appearance of fluorescein-stained spots on 1^{st} day of the modeling

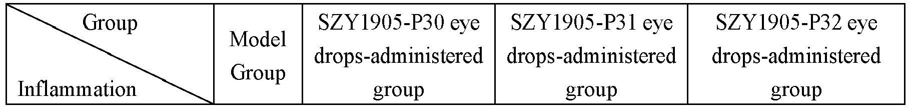

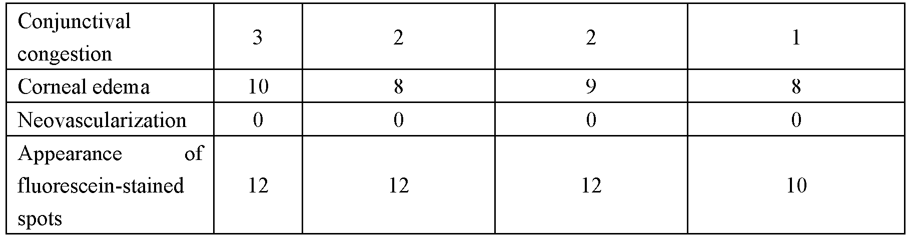

Number of New Zealand rabbit eyes with conjunctival congestion, corneal edema, neovascularization, and appearance of fluorescein-stained spots on 7^{th} day of the modeling

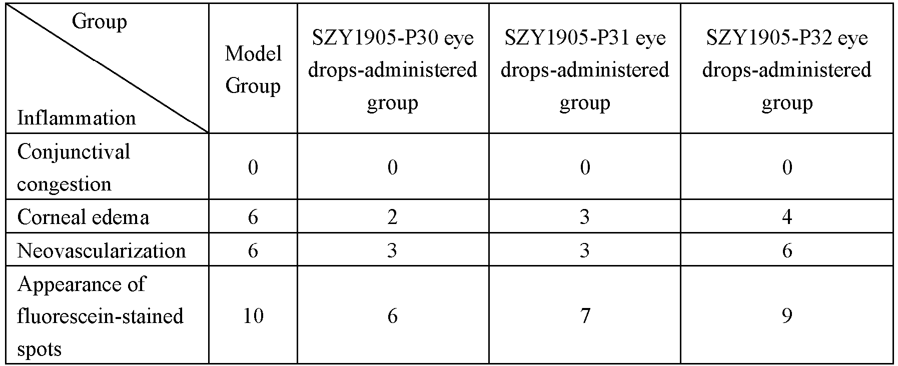

Number of New Zealand rabbit eyes with conjunctival congestion, corneal edema, neovascularization, and appearance of fluorescein-stained spots on 14^{th} day of the modeling

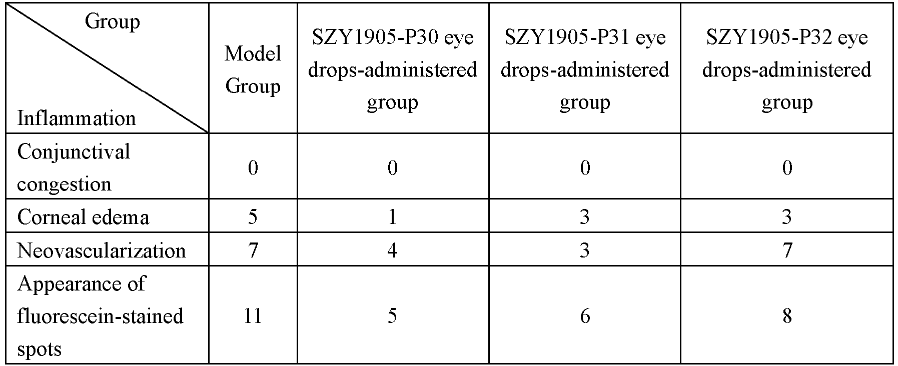

### (2) Fluorescein staining score

### Scoring Criteria:

0- No stained spots on corneal epithelium
1 Stained spots on corneal epithelium less than 30
2- Stained spots on corneal epithelium less than half of the area of the corneal
3 Stained spots on corneal epithelium greater than half of the area of the corneal, or appearance of large areas of staining

### (3) Neovascularization score

0- No new vessels formed in the cornea
1- A small number of new vessels formed in the cornea
2- A large number of new vessels formed in the cornea
3 New vessels formed all over the cornea

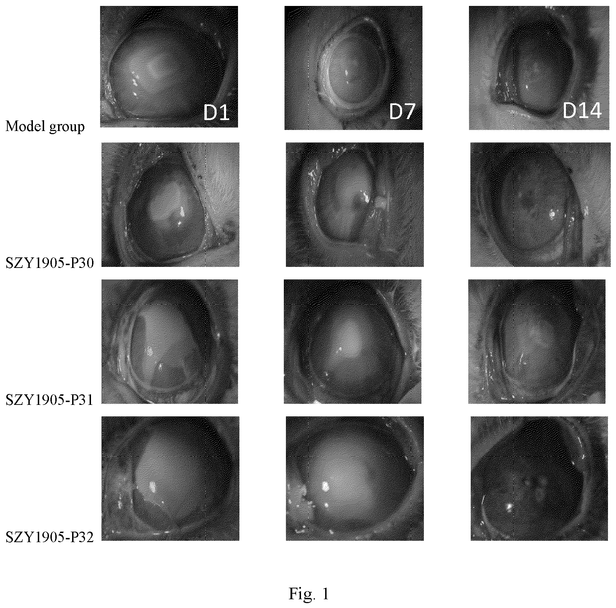

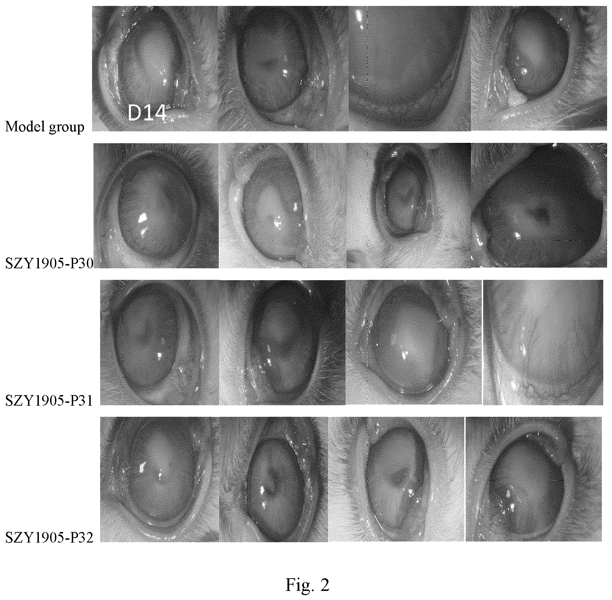

Photographs for fluorescein staining in each of the treatment groups in Example 6 are shown in Fig. 1, and photographs for neovascularization in each of the treatment groups in Example 6 are shown in Fig. 2. As can be seen from the figures, on the 7^{th} day after the modeling, a small number of new vessels start to form in some of the New Zealand rabbits with alkali burn. By comparing the New Zealand rabbits with neovascularization in the four groups, it can be concluded preliminarily that the SZY1905-P30-administered group and the SZY1905-P31-administered group can delay the rapid formation of new blood vessels significantly better than the SZY1905-P32 eye drops-administered group in which new vessels have spread to the entire cornea of the New Zealand rabbits.

## Claims

1. A compound shown in Formula I or a pharmaceutically acceptable salt thereof, 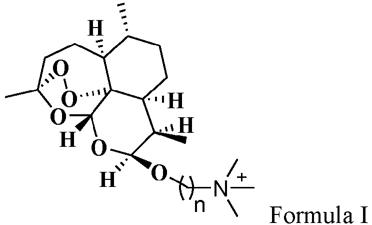 wherein in Formula I, n is an integer from 1 to 15.

2. The compound or the pharmaceutically acceptable salt thereof according to claim 1, wherein n is 3 or 4.

3. The compound or the pharmaceutically acceptable salt thereof according to claim 1 or 2, wherein:
the pharmaceutically acceptable salt of the compound shown in Formula is citrate, trans-butenedioate, salicylate, L-tartrate, fumarate, sodium salt, potassium salt, calcium salt, hydrochloride, ethanoate, nitrate, sulfate, bisulfate, phosphate, biphosphate, acetate, oxalate, lactate, lysine, or aspartate.

4. A method of preparing the compound according to any one of claims 1 to 3, comprising the following steps:
1) dissolving 3-dimethylamino-1-alkyl alcohol in acetonitrile, and adding iodomethane drop-wise, followed by stirring for a reaction to obtain a quaternary ammonium salt intermediate; and
2) dispersing dihydroartemisinin in dichloromethane, followed by successively adding the quaternary ammonium salt intermediate and an catalytic amount of boron trifluoride ether for a reaction to obtain the compound shown in Formula I.

5. Use of the compound or the pharmaceutically acceptable salt thereof according to any one of claims 1 to 3 in preparation of a drug for prevention and/or treatment of an ocular tissue disease.

6. The use according to claim 5, wherein the ocular tissue disease is ocular tissue inflammation or ocular tissue macular degeneration.

7. Use of the compound shown in Formula I or the pharmaceutically acceptable salt thereof in preparation of an inhibitor for prevention and/or treatment of corneal neovascularization.

8. A drug or a pharmaceutical composition for prevention and/or treatment of an ocular tissue disease, comprising the compound or the pharmaceutically acceptable salt thereof according to any one of claims 1 to 3.

9. The use according to claim 8, wherein the ocular tissue disease is ocular tissue inflammation or ocular tissue macular degeneration.

10. An inhibitor for prevention and/or treatment of corneal neovascularization, wherein an active ingredient of the inhibitor comprises the compound or the pharmaceutically acceptable salt thereof according to any one of claims 1 to 3.
